# EUROPÄISCHE PATENTSCHRIFT

(11) **EP 0 707 020 B1**
(45) Veröffentlichungstag und Bekanntmachung des Hinweises auf die Patenterteilung: **23.08.2000**
(21) Anmeldenummer: 95115511.8
(22) Anmeldetag: 02.10.1995
(51) Int. Cl.: C08G 61/00, C09K 11/06, H05B 33/14, C07C 25/22, C07C 69/76

(54) **Konjugierte Polymere mit Spirozentren und ihre Verwendung als Elektrolumineszenzmaterialien**
Conjugated polymers with a spiro atom and their use as electroluminescent materials
Polymères conjugués spiranniques et leur utilisation comme matériaux électroluminescents

(30) Priorität: 14.10.1994 DE 4436773
(43) Veröffentlichungstag der Anmeldung: 17.04.1996
(73) Patentinhaber: Axiva GmbH, 65926 Frankfurt am Main (DE)
(72) Erfinder: Kreuder, Willi, Dr., D-55126 Mainz (DE); Lupo, Donald, Dr., D-60316 Frankfurt (DE); Salbeck, Josef, Dr., D-65719 Hofheim (DE); Schenk, Hermann, Dr., D-65719 Hofheim (DE); Stehlin, Thomas, Dr., D-65830 Kriftel (DE)

(56) Entgegenhaltungen:
- GB-A- 998 250

## Beschreibung

Es besteht ein höher industrieller Bedarf an großflächigen Festkörper-Lichtquellen für eine Reihe von Anwendungen, überwiegend im Bereich von Anzeigeelementen, der Bildschirmtechnologie und der Beleuchtungstechnik. Die an diese Lichtquellen gestellten Anforderungen können zur Zeit von keiner der bestehenden Technologien völlig befriedigend gelöst werden.

Als Alternative zu herkömmlichen Anzeige- und Beleuchtungselementen, wie Glühlampen, Gasentladungslampen und nicht selbstleuchtenden Flüssigkristallanzeigeelementen, sind bereits seit einiger Zeit Elektrolumineszenz(EL)materialien und -vorrichtungen, wie lichtemittierende Dioden (LED), in Gebrauch.

Neben anorganischen sind seit etwa 30 Jahren auch niedermolekulare organische Elektrolumineszenzmaterialien und -vorrichtungen bekannt (siehe z.B. US-A-3,172,862). Bis vor kurzem waren aber solche Vorrichtungen in ihrer praktischen Verwendbarkeit stark eingeschränkt.

In WO 90/13148 und EP-A 0 443 861 sind Elektrolumineszenzvorrichtungen beschrieben, die einen Film aus einem konjugierten Polymer als lichtemittierende Schicht (Halbleiterschicht) enthalten. Solche Vorrichtungen bieten zahlreiche Vorteile wie die Möglichkeit, großflächige, flexible Displays einfach und kostengünstig herzustellen. Im Gegensatz zu Flüssigkristalldisplays sind Elektrolumineszenzdisplays selbstleuchtend und benötigen daher keine zusätzliche rückwärtige Beleuchtungsquelle.

Eine typische Vorrichtung nach WO 90/13148 besteht aus einer lichtemittierenden Schicht in Form eines dünnen, dichten Polymerfilms (Halbleiterschicht), der wenigstens ein konjugiertes Polymer enthält. Eine erste Kontaktschicht steht in Kontakt mit einer ersten Oberfläche, eine zweite Kontaktschicht mit einer weiteren Oberfläche der Halbleiterschicht. Der Polymerfilm der Halbleiterschicht hat eine genügend geringe Konzentration von extrinsischen Ladungsträgern, so daß beim Anlegen eines elektrischen Feldes zwischen den beiden Kontaktschichten Ladungsträger in die Halbleiterschicht eingebracht werden, wobei die eine Kontaktschicht positiv gegenüber der anderen wird, und die Halbleiterschicht Strahlung aussendet. Die in solchen Vorrichtungen verwendeten Polymere sind konjugiert. Unter konjugiertem Polymer versteht man ein Polymer, das ein delokalisiertes Elektronensystem entlang der Hauptkette besitzt. Das delokalisierte Elektronensystem verleiht dem Polymer Halbleitereigenschaften und gibt ihm die Möglichkeit, positive und/oder negative Ladungsträger mit hoher Mobilität zu transportieren.

In WO 90/13148 wird als polymeres Material für die lichtemittierende Schicht Poly(p-phenylenvinylen) verwendet, und es wird vorgeschlagen, die Phenylgruppe in einem solchen Material durch ein heterocyclisches oder ein kondensiertes carbocyclisches Ringsystem zu ersetzen. Daneben wird auch Poly(p-phenylen), PPP, als elektrolumineszierendes Material verwendet (G. Grem, G. Leditzky, B. Ullrich, G. Leising, Synth. Met. 1992, 51, Seite 383).

Obwohl mit diesen Materialien gute Ergebnisse erzielt wurden, ist beispielsweise die Farbreinheit noch unbefriedigend. Weiterhin ist es mit den bisher bekannten Polymeren kaum möglich, eine blaue oder weiße Emission zu erzeugen.

Da zudem die Entwicklung von Elektrolumineszenzmaterialien, insbesondere auf Grundlage von Polymeren, noch in keiner Weise als abgeschlossen betrachtet werden kann, sind die Hersteller von Beleuchtungs- und Anzeigevorrichtungen an den unterschiedlichsten Elektrolumineszenzmaterialien für solche Vorrichtungen interessiert.

Dies liegt unter anderem auch daran, weil erst das Zusammenwirken der Elektrolumineszenzmaterialien mit den weiteren Bauteilen der Vorrichtungen Rückschlüsse auf die Qualität auch des Elektrolumineszenzmaterials zuläßt.

Aufgabe der vorliegenden Erfindung war es daher, neue Elektrolumineszenzmaterialien bereitzustellen, die bei Verwendung in Beleuchtungs- oder Anzeigevorrichtung geeignet sind, das Eigenschaftsprofil dieser Vorrichtungen zu verbessern.

Es wurde nun überraschend gefunden, daß konjugierte Polymere, deren Wiederholeinheiten ein 9,9'-Spirobifluorengerüst enthalten, neben einer verbesserten Löslichkeit in organischen Solventien und verbesserten Filmbildungseigenschaften insbesondere auch gute Elektro- und Photolumineszenz mit einer hohen Farbreinheit aufweisen.

Spiroverbindungen sind Verbindungen in denen zwei Ringsysteme durch ein einziges, vierbindiges Atom verknüpft sind. Dieses Atom wird als Spiroatom bezeichnet, wie in Handbook of Chemistry and Physics 62^{nd} ed. (1981-2), CRC Press, S. C-23 bis C-25 ausgeführt ist.

Verbindungen, bei denen zwei Polymere über ein einziges Spiroatom verknüpft sind, sind beispielsweise in US-A 5,026,894 und bei J. M. Tour et al., J. Am. Chem. Soc. 1990, 112, 5662; J. M. Tour et al., J. Am. Chem. Soc. 1991, 113, 7064 und J. M. Tour et al., Polym. Prepr. 1990, 408 als Materialien für molekulare Elektronik vorgeschlagen. Eine mögliche Eignung solcher Verbindungen als Elektrolumineszenzmaterialien läßt sich daraus nicht ableiten. Gegenstand der Erfindung sind daher konjugierte Polymere gemäß Anspruch 1.

Die erfindungsgemäßen Polymere zeichnen sich insbesondere durch eine hohe Farbeinheit der Emission aus.

Polymer bedeutet im Sinne der Erfindung eine Verbindung, deren Elektrolumineszenzspektrum bei Anfügen weiterer Wiederholeinheiten im wesentlichen gleich bleibt.

Die erfindungsgemäßen Polymere weisen im allgemeinen 2 bis 1000, vorzugsweise 2 bis 500, besonders bevorzugt 2 bis 100, Wiederholeinheiten der Formel (I) auf.

Bevorzugt sind weiterhin solche Polymere, bei denen die Symbole und Indizes in der allgemeinen Formel (I) folgende Bedeutungen haben:
- S: ist gleich oder verschieden R¹, R², R³ und/oder R⁴;
- A, B: sind gleich oder verschieden 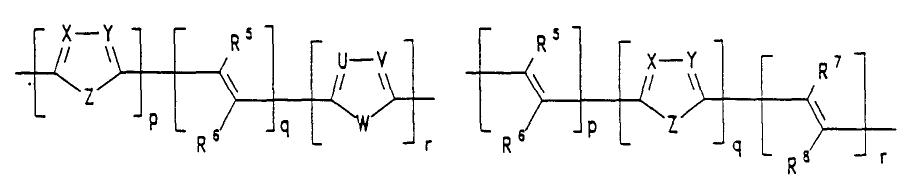 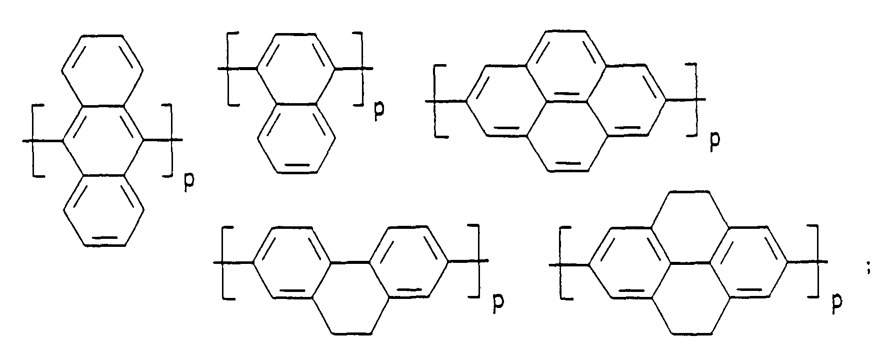
- X, Y, U, V: sind gleich oder verschieden CR⁵, N;
- Z, W: sind gleich oder verschieden -O-, -S-, -NR⁵-, -CR⁵R⁶-, -CR⁵ =CR⁶-, -CR⁵=N-;
- p, q, r: sind gleich oder verschieden 0, 1 bis 5;
- R¹, R², R³, R⁴, R⁵, R⁶, R⁷, R⁸: sind gleich oder verschieden
H, eine geradkettige oder verzweigte Alkyl, Alkoxy oder Estergruppe mit 1 bis 22 C-Atomen, Aryl- und/oder Aryloxygruppen, vorzugsweise Phenyl- und/oder Phenyloxygruppen, wobei der Aromat mit C₁-C₂₂-Alkyl, C₁-C₂₂-Alkoxy, Br, Cl, F, CN, und/oder NO₂ substituiert sein kann, Br, Cl, F, CN, NO₂, CF₃.

Besonders bevorzugt sind Polymere, bei denen die Symbole und Indizes in der Formel (I) folgende Bedeutungen haben:
- A, B: sind gleich oder verschieden 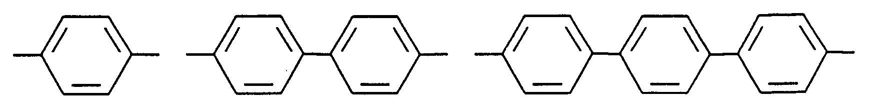 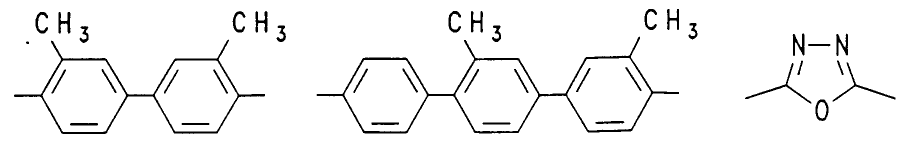 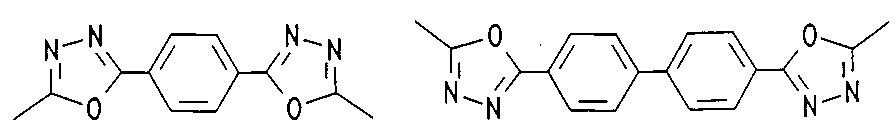 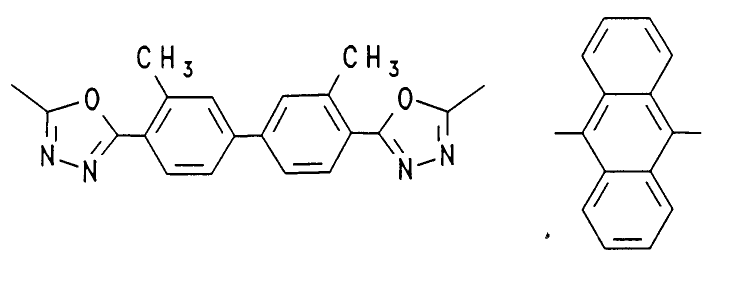
- m, n: sind gleich oder verschieden 0 oder 1;
- C, D: sind gleich oder verschieden 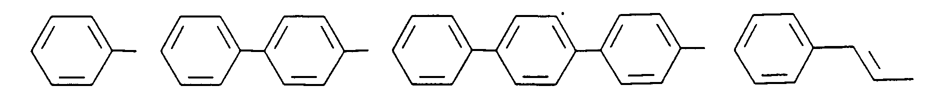 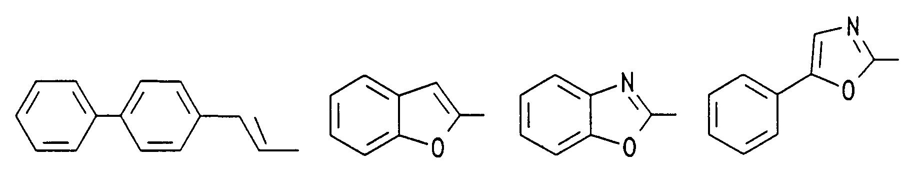 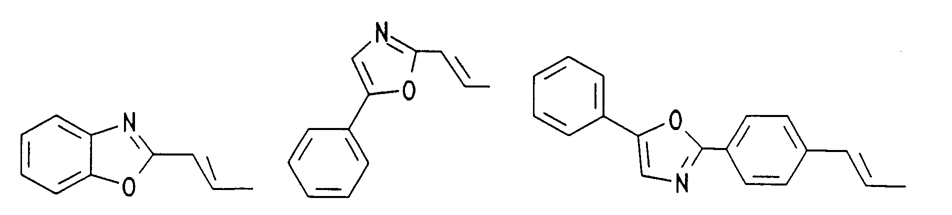 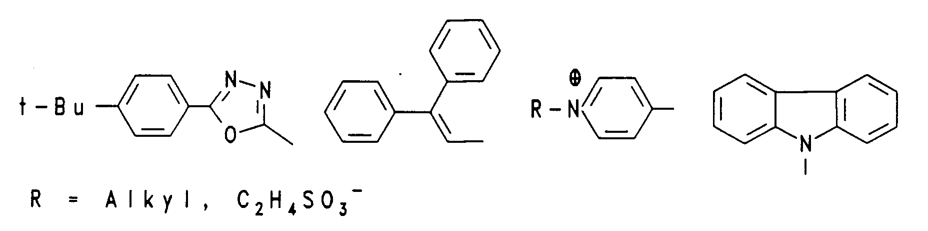 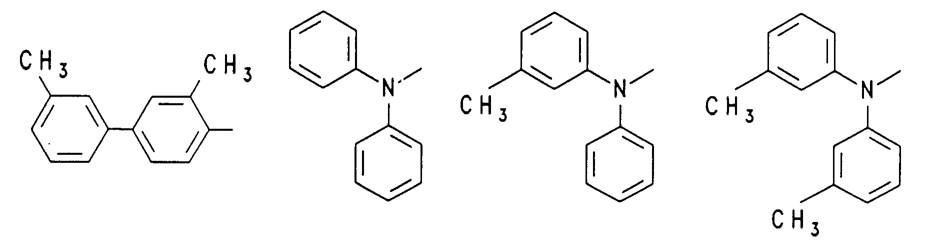
Ganz besonders bevorzugt sind Polymere, bei denen die Symbole und Indizes in der Formel (I) folgende Bedeutungen haben:
- A, B: sind gleich oder verschieden 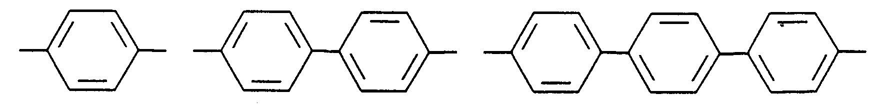 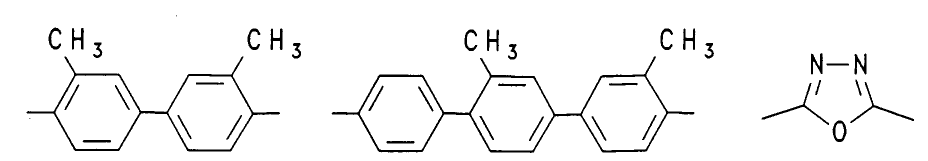 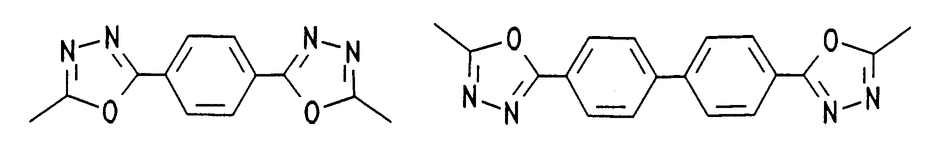 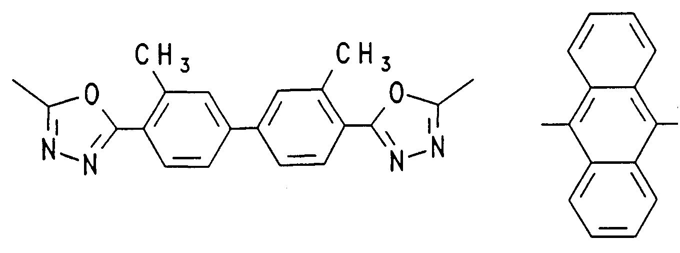
- m + n: ist 0 oder 1;
- C, D: sind gleich oder verschieden 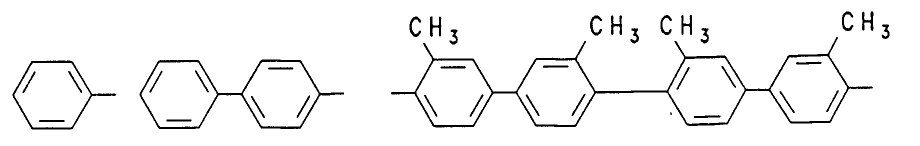 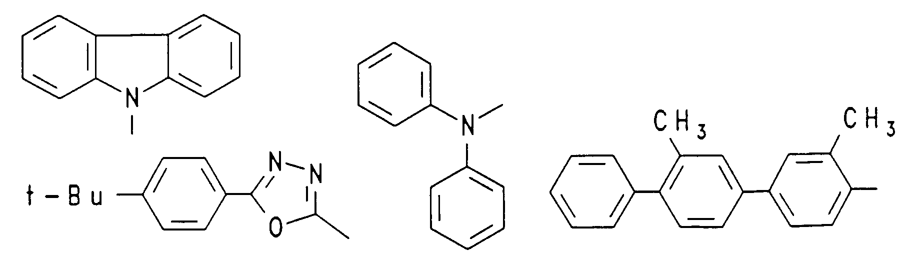
Die erfindungsgemäßen Polymere sind Homo- oder Copolymere, d.h., daß sie auch unterschiedliche Wiederholeinheiten der Formel (I) aufweisen können.

Die erfindungsgemäßen Polymere zeichnen sich weiterhin durch eine beträchtlich gesteigerte Löslichkeit in organischen Solventien und gute Filmbildungseigenschaften aus. Dadurch wird die Herstellung von Elektrolumineszenzvorrichtungen erleichtert und deren Lebensdauer erhöht. Darüber hinaus erlaubt die kovalent gebundene Anordnung der Substituenten über die Spirozentren, senkrecht zur konjugierten Hauptkette, einen molekularen Aufbau in der Weise, daß ohne Störung der Konjugation in der Hauptkette bestimmte Eigenschaften eingestellt werden können. So kann die Polymerkette z.B. Ladungstransport- oder Ladungsinjektionseigenschaften besitzen, während die Substituenten lichtemittierende Eigenschaften besitzen. Die Emissionseigenschaften der erfindungsgemäß eingesetzten Verbindungen können durch die Wahl geeigneter Substituenten über den ganzen Bereich des sichtbaren Spektrums eingestellt werden. Die durch die kovalente Anknüpfung fixierte räumliche Nähe der beiden Hälften ist dabei günstig für die Energieübertragung (siehe z.B. B. Liphardt, W. Lüttke Liebigs Ann. Chem. 1981, 1118).

Die erfindungsgemäßen Polymere sind zur Erzielung blauer Elektrolumineszenz gut geeignet.

Die Herstellung der erfindungsgemäßen Polymere kann nach an sich literaturbekannten Methoden, wie sie in Standardwerken zur Organischen Synthese, z.B. Houben-Weyl, Methoden der Organischen Chemie, Georg-Thieme-Verlag, Stuttgart, beschrieben werden, erfolgen.

Die Herstellung erfolgt dabei unter Reaktionsbedingungen, die für die genannten Umsetzungen bekannt und geeignet sind. Dabei kann auch von an sich bekannten, hier nicht näher erwähnten Varianten Gebrauch gemacht werden.

Als Ausgangsverbindungen für die Herstellung der erfindungsgemäßen Polymere kommen im allgemeinen Monomere mit einem 9,9'-Spirobifluoren-Zentrum zum Einsatz, die in 2,7- bzw. gegebenenfalls 2',7'-Position substituiert sind.

Methoden zur Synthese dieser Monomere beruhen z.B. auf der Synthese des 9,9'-Spirobifluorens, z.B. aus 2-Brombiphenyl und Fluorenon über eine Grignardsynthese, wie sie von R. G. Clarkson, M. Gomberg, J. Am. Chem. Soc. 1930, 52, Seite 2881 beschrieben ist, welches anschließend weiter in geeigneter Weise substituiert wird.

Funktionalisierungen von 9,9'-Spirobifluoren sind beispielsweise beschrieben in J. H. Weisburger, E. K. Weisburger, F. E. Ray, J. Am. Chem. Soc. 1959, 72, 4253; F. K. Sutcliffe, H. M. Shahidi, D. Paterson, J. Soc. Dyers Colour 1978, 94, 306; und G. Haas, V. Prelog, Helv. Chim. Acta 1969, 52, 1202.

Wesentlich günstiger erhält man das gewünschte Substitutionsmuster des 9,9'-Spirobifluoren-Monomers, wenn die Spiroverknüpfung bereits ausgehend von geeignet substituierten Edukten erfolgt, z.B. mit 2,7-difunktionalisierten Fluorenonen, und die noch freien 2',7'-Positionen nach Aufbau des Spiroatoms dann gegebenenfalls weiter funktionalisiert werden (z.B. durch Halogenierung oder Acylierung, mit anschließender C-C-Verknüpfung nach Umwandlung der Acetylgruppen in Aldehydgruppen, oder durch Heterocyclenaufbau nach Umwandlung der Acetylgruppen in Carbonsäuregruppen).

Die weitere Funktionalisierung kann nach an sich literaturbekannten Methoden erfolgen, wie sie in Standardwerken zur Organischen Synthese, z.B Houben-Weyl, Methoden der Organischen Chemie, Georg-Thieme Verlag, Stuttgart und in den entsprechenden Bänden der Serie "The Chemistry of Heterocyclic Compounds" von A. Weissberger und E. C. Taylor (Herausgeber) beschrieben sind.

Für die Synthese der Gruppen A, B, C, D sei beispielsweise verwiesen auf DE-A 23 44 732, 24 50 088, 24 29 093, 25 02 904, 26 36 684, 27 01 591 und 27 52 975 für Verbindungen mit 1,4-Phenylen-Gruppen DE-A 26 41 724 für Verbindungen mit Pyrimidin-2,5-diyl-Gruppen; DE-A 40 26 223 und EP-A 03 91 203 für Verbindungen mit Pyridin-2,5-diyl-Gruppen; DE-A 32 31 462 für Verbindungen mit Pyridazin-3,6-diyl-Gruppen; N. Miyaura, T. Yanagi und A. Suzuki in Synthetic Communications 1981, 11, 513 bis 519, DE-C-3 930 663, M. J. Sharp, W. Cheng, V. Snieckus in Tetrahedron Letters 1987, 28, 5093; G. W. Gray in J.Chem.Soc. Perkin Trans II 1989, 2041 und Mol. Cryst. Liq. Cryst. 1989, 172, 165, Mol. Cryst. Liq. Cryst. 1991, 204, 43 und 91; EP-A 0 449 015; WO 89/12039; WO 89/03821; EP-A 0 354 434 für die direkte Verknüpfung von Aromaten und Heteroaromaten;

Die Herstellung disubstituierter Pyridine, disubstituierter Pyrazine, disubstituierter Pyrimidine und disubstituierter Pyridazine findet sich beispielsweise in den entsprechenden Bänden der Serie "The Chemistry of Heterocyclic Compounds" von A. Weissberger und E. C. Taylor (Herausgeber).

Ausgehend von den oben angegebenen Monomeren ist die Polymerisation zu den erfindungsgemäßen Polymeren nach mehreren Methoden möglich.

Beispielsweise können Derivate des 9,9'-Spirobifluorens oxidativ (z.B. mit FeCl₃, siehe u.a. P. Kovacic, N. B. Jones, Chem. Ber. 1987, 87, 357 bis 379; M. Weda, T. Abe, H. Awano, Macromolecules 1992, 25, 5125) oder elektrochemisch (siehe z.B. N. Saito, T. Kanbara, T. Sato, T. Yamamoto, Polym. Bull. 1993, 30, 285) polymerisiert werden.

Ebenso können die erfindungsgemäßen Polymere aus 2,7-difunktionalisierten 9,9'-Spirobifluoren-Derivaten hergestellt werden.
Dihalogenaromaten lassen sich unter Kupfer/Triphenylphosphan (siehe z.B. G. W. Ebert, R. D. Rieke, J. Org. Chem. 1988, 53, 44829 oder Nickel/Triphenylphosphan-Katalyse (siehe z.B. H. Matsumoto, S. Inaba, R. D. Rieke, J. Org. Chem. 1983, 48, 840) polymerisieren.

Aromatische Diboronsäuren und aromatische Dihalogenide oder gemischte aromatische Halogen-Boronsäuren lassen sich unter Palladiumkatalyse durch Kupplungsreaktionen polymerisieren (siehe z.B. M. Miyaura, T. Yanagi, A. Suzuki, Synth. Commun. 1981, 11, 513; R. B. Miller, S. Dugar, Organometallics 1984, 3, 1261).

Aromatische Distannane lassen sich, z.B. wie bei J. K. Stille, Angew. Chem. Int. Ed. Eng. 1986, 25, 508 angegeben, unter Palladiumkatalyse polymerisieren.

Weiterhin können die oben erwähnten Dibromverbindungen in die Dilithio- oder Digrignardverbindungen übergeführt werden, die dann mit weiterer Dibromverbindung mittels CuCl₂ (siehe z.B. G. Wittig, G. Klar, Liebigs Ann. Chem. 1967, 704, 91; H. A. Stabb, F. Bunny, Chem. Ber. 1967, 100, 293; T. Kaufmann, Angew. Chem. 1974, 86, 321 bis 354) oder durch Elektronentransfer ungesättigter 1,4-Dihalogenverbindungen (siehe z.B. S. K. Taylor, S. G. Bennett, K. J. Harz, L. K. Lashley, J. Org. Chem. 1981, 46, 2190) polymerisiert werden.

Die Synthese der erfindungsgemäßen Polymere kann aber auch durch Polymerisation eines 2,7-difunktionalisierten 9,9'-Spirobifluorenderivates mit einer weiteren, geeignet difunktionalisierten Verbindung erfolgen.

So kann z.B. 2,7-Dibrom-9,9'-spirobifluoren mit 4,4'-Biphenylylbisboronsäure polymerisiert werden. Auf diese Weise ist gleichzeitig mit dem Polymerisationsschritt der Aufbau verschiedener heterocyclischer Einheiten möglich, wie z.B. die Bildung von Oxadiazoleinheiten aus difunktionellen Carbonsäurehalogeniden und difunktionellen Carbonsäurehydraziden bzw. aus der entsprechenden Dicarbonsäure und Hydrazinsulfat (B. Schulz, E. Leibnitz, Acta Polymer 1992, 43, Seite 343; JP-A 05/178990, oder alternativ aus Dicarbonsäurehalogeniden und Bistetrazolen (C. A. Abshire, C. S. Marvel, Makromol. Chem. 1961, 44 bis 46, Seite 388).

Zur Herstellung von Copolymeren können beispielsweise unterschiedliche Verbindungen der Formel (I) gemeinsam polymerisiert werden.

Die Aufarbeitung erfolgt nach bekannten, dem Fachmann geläufigen Methoden, wie sie beispielsweise bei D. Braun, H. Cherdron, W. Kern, Praktikum der makromolekularen organischen Chemie, 3. Aufl. Hüthig Verlag, Heidelberg, 1979, S. 87 ff. oder R. J. Young, P. A. Lovell, Introduction to Polymers, Chapman & Hall, London 1991 beschrieben sind. Beispielsweise kann man die Reaktionsmischung filtrieren, mit wäßriger Säure verdünnen, extrahieren und das nach Trocknen und Abziehen des Lösungsmittels erhaltene Rohprodukt durch Umfällen weiter reinigen.

Endständige Bromatome können beispielsweise mit LiAlH₄ reduktiv entfernt werden (siehe z.B. J. March, Advanced Organic Chemistry, 3. Aufl. McGraw-Hill, S. 510).

Die erfindungsgemäßen Polymere können als Elektrolumineszenzmaterialien Verwendung finden.

Gegenstand der Erfindung ist daher auch die Verwendung von Polymeren, enthaltend Wiederholeinheiten der Formel (I), als Elektrolumineszenzmaterial.

Als Elektrolumineszenzmaterial im Sinne der Erfindung gelten Stoffe, die als aktive Schicht in einer Elektrolumineszenzvorrichtung Verwendung finden können. Aktive Schicht bedeutet, daß die Schicht befähigt ist, bei Anlegen eines elektrischen Feldes Licht abzustrahlen (lichtemittierende Schicht) und/oder daß sie die Injektion und/oder den Transport der positiven und/oder negativen Ladungen verbessert (Ladungsinjektions- oder Ladungstransportschicht).

Gegenstand der Erfindung ist daher auch ein Elektrolumineszenzmaterial, enthaltend ein oder mehrere Polymere, die Wiederholeinheiten der Formel (I) enthalten.

Üblicherweise enthält das erfindungsgemäße Elektrolumineszenzmaterial ein oder mehrere erfindungsgemäßen Polymere entweder als Hauptkomponente, d.h. zu größer als 50 Gew.-%, oder als Additiv.

Um als Elektrolumineszenzmaterialien Verwendung zu finden, werden die erfindungsgemäßen Polymere im allgemeinen nach bekannten, dem Fachmann geläufigen Methoden, wie Gießen (Casting), Eintauchen (Dipping) oder Aufschleudern (Spincoating), in Form eines Films auf ein Substrat aufgebracht.

Weiterhin Gegenstand der Erfindung ist daher auch ein Verfahren zur Herstellung eines Elektrolumineszenzmaterials, dadurch gekennzeichnet, daß man ein Polymer, enthaltend Wiederholeinheiten der Formel (I), in Form eines Films auf ein Substrat aufbringt.

Gegenstand der Erfindung ist zudem eine Elektrolumineszenzvorrichtung mit einer oder mehreren aktiven Schichten, wobei mindestens eine dieser aktiven Schichten ein oder mehrere erfindungsgemäße Polymere enthält. Die aktive Schicht kann beispielsweise eine lichtemittierende Schicht und/oder eine Transportschicht und/oder eine Ladungsinjektionsschicht sein.

Der allgemeine Aufbau solcher Elektrolumineszenzvorrichtungen ist beispielsweise in US 4,539,507 und US 5,151,629 beschrieben. Polymere enthaltende Elektrolumineszenzvorrichtungen sind beispielsweise in WO 90/13148 oder EP-A 0 443 861 beschrieben.

Sie enthalten üblicherweise eine elektrolumineszierende Schicht zwischen einer Kathode und einer Anode, wobei mindestens eine der Elektroden transparent ist. Zusätzlich kann zwischen der elektrolumineszierenden Schicht und der Kathode eine Elektroneninjektions- und/oder Elektronentransportschicht eingebracht sein und/oder zwischen der elektrolumineszierenden Schicht und der Anode eine Lochinjektions- und/oder Lochtransportschicht eingebracht sein. Als Kathode können z.B. Ca, Mg, Al, In, Mg/Ag dienen. Als Anode können z.B. Au oder ITO (Indiumoxid/Zinnoxid) auf einem transparentem Substrat, z.B. aus Glas oder einem transparenten Polymer, dienen.

Im Betrieb wird die Kathode auf negatives Potential gegenüber der Anode gesetzt. Dabei werden Elektronen von der Kathode in die Elektroneninjektionsschicht/Elektronentransportschicht bzw. direkt in die lichtemittierende Schicht injiziert. Gleichzeitig werden Löcher von der Anode in die Lochinjektionsschicht/ Lochtransportschicht bzw. direkt in die lichtemittierende Schicht injiziert.

Die injizierten Ladungsträger bewegen sich unter dem Einfluß der angelegten Spannung durch die aktiven Schichten aufeinander zu. Dies führt an der Grenzfläche zwischen Ladungstransportschicht und lichtemittierender Schicht bzw. innerhalb der lichtemittierenden Schicht zu Elektronen/Loch-Paaren, die unter Aussendung von Licht rekombinieren.
Die Farbe des emittierten Lichtes kann durch das als lichtemittierende Schicht verwendete Material variiert werden.

Elektrolumineszenzvorrichtungen finden Anwendung z.B. als selbstleuchtende Anzeigeelemente, wie Kontrollampen, alphanumerische Displays, Hinweisschilder, und in optoelektronischen Kopplern.

Die Erfindung wird durch die Beispiele näher erläutert, ohne sie dadurch beschränken zu wollen.

### Beispiele

### A Monomersynthesen

### Beispiel 1 Synthese von 2,7-Dibrom-9,9'-spirobifluoren

Ein Grignardreagenz bereitet aus 0,72 g (30 mmol) Magnesiumspänen und 5,1 ml (30 mmol) 2-Brombiphenyl in 15 ml Diethylether wird im Verlauf von 2 Stunden unter Rühren (im Ultraschallbad) zu einer siedenden Suspension von 10,0 g (29,6 mmol) 2,7-Dibrom-9-fluorenon in 100 ml trockenem Diethylether getropft. Nach beendeter Zugabe wird 3 Stunden weiter gekocht. Nach Abkühlung über Nacht wird der ausgefallene Niederschlag abgesaugt und mit kaltem Ether gewaschen. Der abgesaugte Magnesiumkomplex wird in einer Lösung von 15 g Ammoniumchlorid in 250 ml Eiswasser hydrolysiert. Nach 1 Stunde wird das gebildete 9-(2-Biphenylyl)-2,7-dibrom-9-fluorenol abgesaugt, mit Wasser gewaschen und trockengesaugt. Das getrocknete Fluorenol wird für die Ringschlußreaktion in 100 ml Eisessig, nach Zugabe von 3 Tropfen konzentrierter Salzsäure 6 Stunden gekocht. Man läßt über Nacht kristallisieren, saugt das gebildete Produkt ab und wäscht mit Eisessig und Wasser. Ausbeute: 11 g (77 %) 2,7-Dibrom-9,9'-spirobifluoren. Zur weiteren Reinigung kann aus THF umkristallisiert werden.
¹H-NMR (CDCl₃, ppm): 6,73 (d, J = 7,63 Hz, 2 H, H-1',8'); 6,84 (d, J = 1,83 Hz, 2 H, H-1,8); 7,15 (td, J = 7,63, 1,22 Hz., 2 H, H-2',7'); 7,41 (td, J = 7,63, 1,22 Hz, 2 H, H-3',6'); 7,48 (dd, J = 8,24, 1,83 Hz, 2 H, H-3,6); 7,67 (d, J = 8,24; 2 H; H-4,5); 7,85 (d, J = 7,63, 2 H, H-4',5').

### Beispiel 2 Synthese von 2,7-Dicarbethoxy-9,9'-spirobifluoren

Ein Grignardreagenz bereitet aus 0,97 g (40 mmol) Magnesiumspänen und 9,32 g (6,8 ml, 40 mmol) 2-Brombiphenyl in 50 ml trockenem Diethylether wird im Verlauf von 2 Stunden zu einer siedenden Lösung von 13 g (40 mmol) 2,7-Dicarbethoxy-9-fluorenon in 100 ml trockenem Diethylether getropft. Nach beendeter Zugabe wird 3 Stunden weiter gekocht. Nach Abkühlung über Nacht wird der ausgefallene Niederschlag abgesaugt und mit kaltem Ether gewaschen. Der abgesaugte Magnesiumkomplex wird in einer Lösung von 15 g Ammoniumchlorid in 250 ml Eiswasser hydrolysiert. Nach 1 Stunde wird das gebildete 9-(2-Biphenylyl)-2,7-dicarbethoxy-9-fluorenol abgesaugt, mit Wasser gewaschen und trockengesaugt. Das getrocknete Fluorenol wird für die Ringschlußreaktion in 100 ml Eisessig, nach Zugabe von 3 Tropfen konzentrierter Salzsäure 6 Stunden gekocht. Man läßt über Nacht kristallisieren, saugt das gebildete Produkt ab und wäscht mit Eisessig und Wasser. Ausbeute: 15,1 g (82 %) 2,7-Dicarbethoxy-9,9'-spirobifluoren. Zur weiteren Reinigung kann aus Ethanol umkristallisiert werden.
¹H-NMR (CDCl₃, ppm): 1,30 (t, J = 7,12 Hz, 6 H, Ester-CH₃); 4,27 (q, J = 7,12 Hz, 4 H, Ester-CH₂); 6,68 (d, J = 7,63 Hz, 2 H, H-1', 8'); 7,11 (td, J = 7,48, 1,22 Hz, 2H, H-2', 7'); 7,40 (td, J = 7,48, 1,22 Hz, 4 H, H-1, 8, 3', 6'); 7,89 (dt, J = 7,63, 0,92 Hz, 2 H, H-4', 5'); 7,94 (dd, J = 7,93, 0,6 Hz, 2 H, H-4, 5); 8,12 (dd, J = 7,93, 1,53 Hz, 2 H, H-3, 6).

### B Polymerisationen

### Beispiel 3 Polymerisation von 2,7-Dibrom-9,9'-spirobifluoren mit Ni(0) nach Yamamoto zu Poly-2,7-(9,9'-spirobifluoren)ylen (Polymer 1)

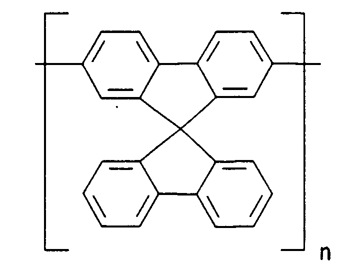

Unter Argon wird eine Lösung von 1,517 g 2,7-Dibrom-9,9'-spirobifluoren in 30 ml trockenem THF bereitet und auf 60°C erwärmt. Die warme Lösung wird rasch, unter Schutzgas zu einer ebenfalls unter Schutzgas am Rückfluß kochenden Mischung aus 825 mg Ni(cod)₂, 470 mg 2,2'-Bipyridyl und 0,4 ml 1,5-Cyclooctadien (COD) in 20 ml trockenem THF gegeben. Die Polymerisation startet sofort, wobei sich die tiefblaue Reaktionsmischung rot färbt. Man läßt 6 Stunden am Rückfluß weiterkochen und kühlt anschließend auf Raumtemperatur ab. Das rotgefärbte Polymer wird abgesaugt und mit THF, sowie verdünnter Salzsäure und Wasser gewaschen.
Durch Extraktion mit 200 ml Chloroform wird eine erste lösliche Polymerfraktion gewonnen (weitere lösliche Fraktionen sind durch Extraktion z.B. mit 1,2-Dichlorethan und 1-Chlornaphthalin gewinnbar), die durch Ausschütteln mit Ethylendiamintetraessigsäure (3x mit Ammoniak auf pH 7 bis 8 eingestellte wäßrige Lösung, 1x pH 3) und nachfolgendem Ausschütteln mit verdünnter Salzsäure und Wasser gereinigt wird. Die getrocknete Chloroformlösung wird auf 10 ml eingeengt und das Polymer durch Eintropfen in 70 ml Methanol ausgefällt. Das erhaltene Polymer ist gelblich gefärbt.
¹H-NMR (CDCl₃, ppm): 6,63 - 6,68 (2H , H-1,8); 6,71- 6,75 (2H, H-1',8'); 7,00 - 7,10 (2H, H-2',7'); 7,21 - 7,38 (4H, H-3,3',6,6'); 7,59-7,70 (2H, H-4',5'); 7,75 -7,82 (2H, H-4,5).

### Beispiel 4 Polymerisation von 2,7-Dibrom-9,9'-spirobifluoren mit 4,4'-Biphenyldiboronsäure zu Poly[2,7-(9,9'spirobifluorenylen)-4,4'-biphenylen](Polymer 2)

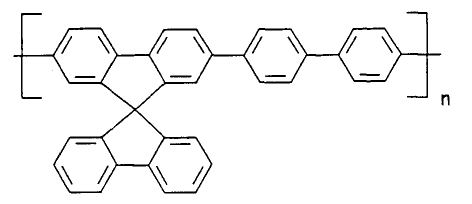

In eine Mischung aus 25 ml THF und 10 ml Ethanol, werden 948 mg (2 mmol) 2,7-Dibromo-9,9'-spirobifluoren und 483 mg (2 mmol) 4,4'-Biphenyldiboronsäure gegeben. Dazu werden 20 ml 1 molare wäßrige Kaliumcarbonatlösung gegeben. Die Mischung wird unter Stickstoff am Rückfluß gekocht und 50 mg Tetrakis(triphenylphosphin)palladium(0), gelöst in 5 ml THF werden zugegeben. Nach 24 Stunden Kochen am Rückfluß wird auf Raumtemperaur abgekühlt. Das gebildete blaßgelbe Polymer wird abgesaugt, mit verdünnter Salzsäure 2 Stunden gekocht und nach erneutem Absaugen mit Wasser säurefrei gewaschen. Durch Extraktion mit 100 ml Chloroform wird eine erste lösliche Polymerfraktion gewonnen (weitere lösliche Fraktionen sind durch Extraktion z.B. mit 1,2-Dichlorethan und 1-Chlornaphthalin gewinnbar).
¹H-NMR (CDCl₃, ppm): 6,75 - 6,85 (2 H); 6,94 (2 H, H-1, 8); 7,05 - 7,15 (2 H); 7,32 - 7,39 (2 H); 7,42 - 7,52 (8 H); 7,61 - 7,68 (2 H); 7,81 - 7,87 (2 H); 7,88 - 7, 92 (2 H).

### Beispiel 5 Photolumineszenzmessung an Poly-2,7-(9,9'-spirobifluoren)ylen (Polymer 1)

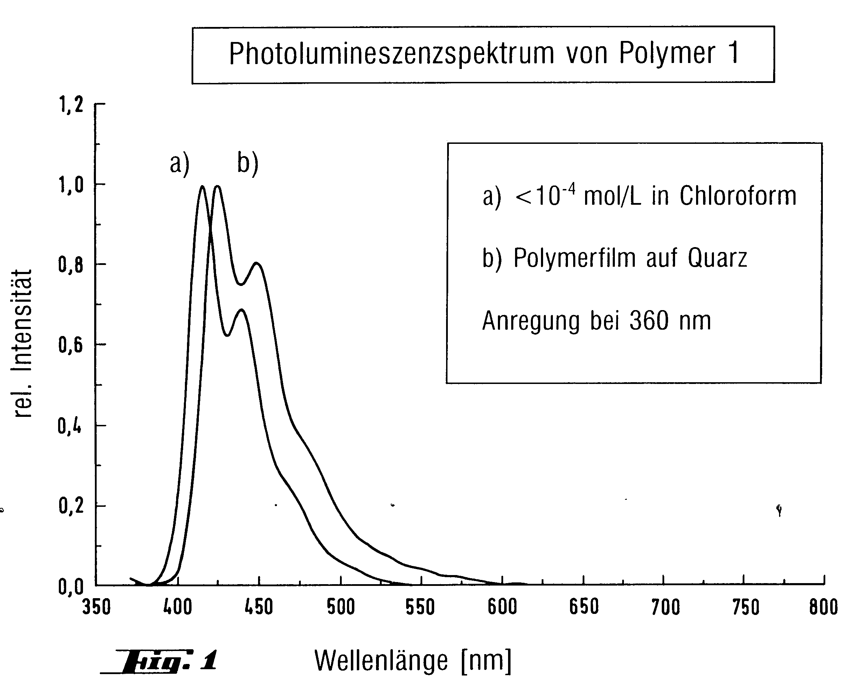

Eine Lösung aus Poly-2,7-(9,9'-spirobifluoren)ylen in Chloroform (5 mg/ml) wird durch Spincoating bei 1000 upm auf einen Quarzträger aufgebracht. Bei Anregung mit Licht einer Wellenlänge < 400 nm zeigt der Polymerfilm homogene blaue Fluoreszenz. Das Fluoreszenzspektrum (Spektrofluorimeter Hitachi F4500, Anregung bei 360 nm) des so hergestellten festen Polymerfilms ist in Abbildung 1 gezeigt. Ein Vergleich mit dem Fluoreszenzspektrum des Polymers 1 in verdünnter Lösung (< 10⁻⁴ mol/L in Chloroform) ergibt für den Film eine bathochrome Verschiebung um 10 nm unter Beibehalt der spektralen Charakteristik der verdünnten Lösung (siehe Abbildung 1).

### Beispiel 6 Photolumineszenzmessung an Poly[2,7-(9,9'-spirobifluorenylen)-4,4'-biphenylen](Polymer 2)

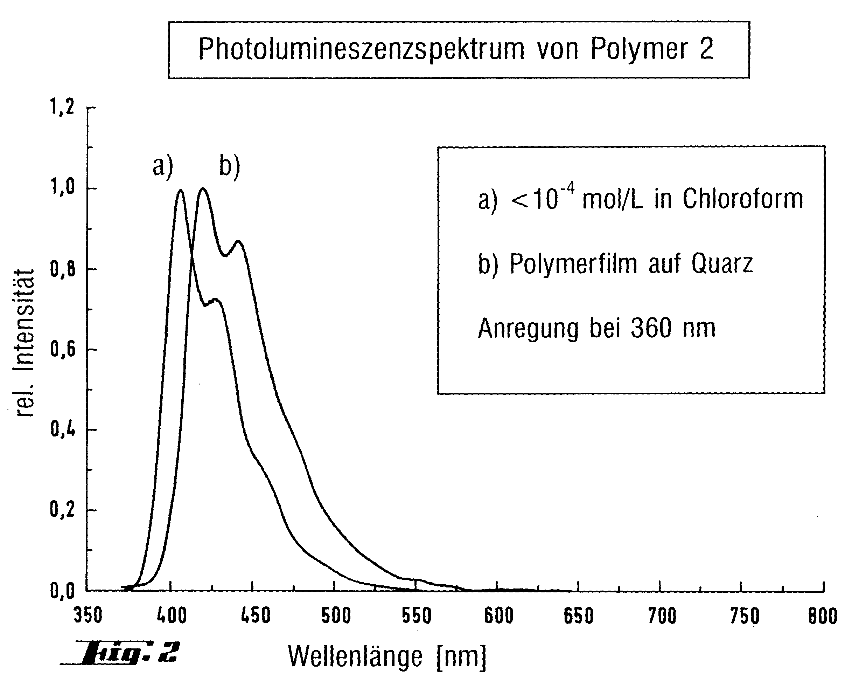

Eine Lösung aus Poly[2,7-(9,9'-spirobifluorenylen)-4,4'-biphenylen] in Chloroform (5 mg/ml) wird durch Spincoating bei 1000 upm auf einen Quarzträger aufgebracht. Bei Anregung mit Licht einer Wellenlänge < 400 nm zeigt der Polymerfilm homogene blaue Fluoreszenz. Das Fluoreszenzspektrum (Spektrofluorimeter Hitachi F4500, Anregung bei 360 nm) des so hergestellten festen Polymerfilms ist in Abbildung 2 gezeigt. Ein Vergleich mit dem Fluoreszenzspektrum des Polymers 2 in verdünnter Lösung (< 10⁻⁴ mol/L in Chloroform) ergibt für den Film eine bathochrome Verschiebung um 15 nm unter Beibehalt der spektralen Charakteristik der verdünnten Lösung (siehe Abbildung 2).

### Elektrolumineszenz-Vorrichtung

Eine Lösung des zu vermessenden Polymers in Chloroform (Konzentration: 15 mg/ml) wird unter Stickstoff durch Spincoating bei 1000 upm auf einen mit ITO (Indium-Zinn-Oxid) beschichteten Glasträger (strukturiert, Streifen 2 mm breit) aufgebracht. Der Glasträger wird über eine Schleuse unter Beibehaltung der Schutzgasatmosphäre in eine Hochvakuum-Bedampfungsanlage überführt. Bei 2x10⁻⁵ mbar werden quer zu den ITO-Streifen unter Verwendung einer Maske Ca-Streifen (2 mm breit, 230 nm dick) auf die Polymerschicht aufgedampft. Die so erhaltene Vorrichtung, ITO/Polymer/Ca, wird in einen Probenhalter gegeben und die Elektroden über Federkontakte mit einer Stromquelle verbunden, wobei ein ITO-Streifen positiv und ein Ca-Streifen negativ gepolt werden. Beim Anlegen einer genügend hohen Spannung, wird an dem entsprechenden Matrixelement eine Elektrolumineszenz beobachtet.

## Patentansprüche

1. Konjugiertes Polymer, enthaltend Wiederholeinheiten der Formel (I), 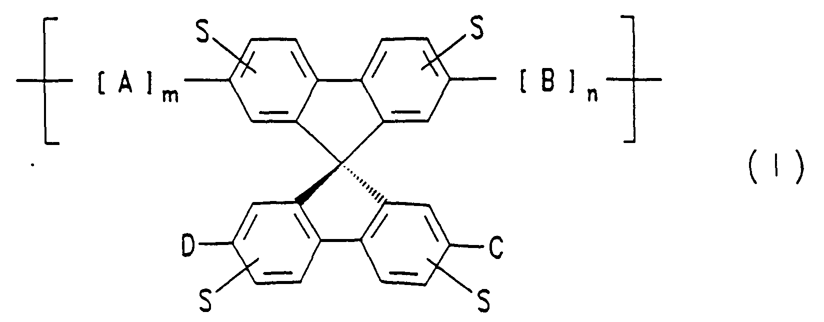 worin die Symbole und Indizes folgende Bedeutungen haben:
A, B sind gleich oder verschieden jeweils eine bis fünfzehn gleiche oder verschiedene Arylen- und/oder Heteroarylen- und/oder Vinylengruppen, die, wie auch das Spirobifluorengerüst, gegebenenfalls substituiert sein können;
C, D sind gleich oder verschieden jeweils eine bis fünfzehn gleiche oder verschiedene Arylen- und/oder Heteroarylen- und/oder Vinylengruppen, die, wie auch das Spirobifluorengerüst, gegebenenfalls substituiert sein können, oder Wasserstoff
S ist gleich oder verschieden H oder ein Substituent;
m, n sind 0 oder 1.

2. Polymer nach Anspruch 1, dadurch gekennzeichnet, daß es 2 bis 1000 Wiederholeinheiten aufweist.

3. Polymer nach Anspruch 1 und/oder 2, dadurch gekennzeichnet, daß die Symbole und Indizes in der allgemeinen Formel (I) folgende Bedeutungen haben:
S ist gleich oder verschieden R¹, R², R³ und/oder R⁴;
A, B sind gleich oder verschieden 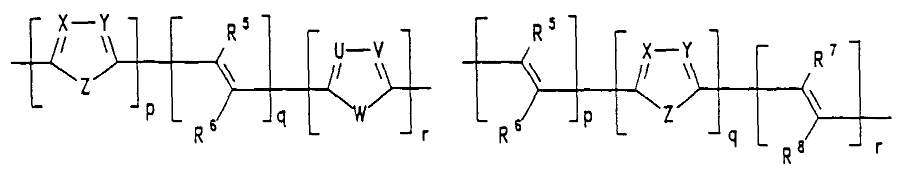 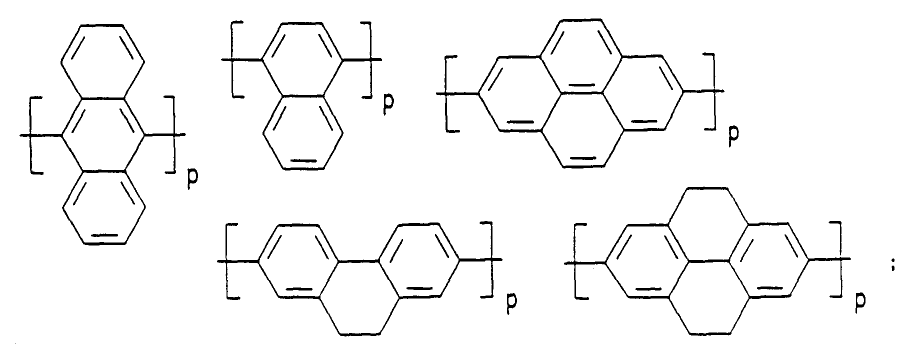
X, Y, U, V sind gleich oder verschieden CR⁵, N;
Z, W sind gleich oder verschieden -O-, -S-, -NR⁵-, -CR⁵R⁶-, -CR⁵ =CR⁶-, -CR⁵=N-;
p, q, r sind gleich oder verschieden 0, 1 bis 5;
R¹, R², R³, R⁴, R⁵, R⁶, R⁷, R⁸ sind gleich oder verschieden
H, eine geradkettige oder verzweigte Alkyl, Alkoxy oder Estergruppe mit 1 bis 22 C-Atomen, Aryl- und/oder Aryloxygruppen, wobei der Aromat mit C₁-C₂₂-Alkyl, C₁-C₂₂-Alkoxy, Br, Cl, F, CN, und/oder NO₂ substituiert sein kann, Br, Cl, F, CN, NO₂, CF₃.

4. Polymer nach einem oder mehreren der vorhergehenden Ansprüche, dadurch gekennzeichnet, daß die Symbole und Indizes in der Formel (I) folgende Bedeutungen haben:
A, B sind gleich oder verschieden 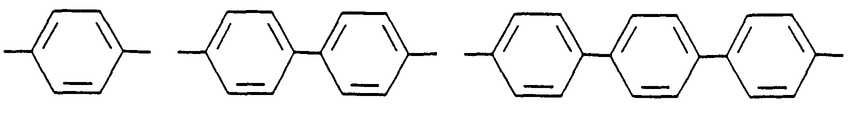 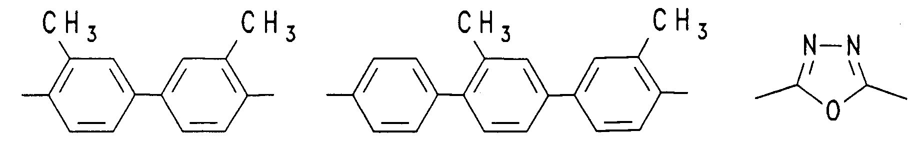 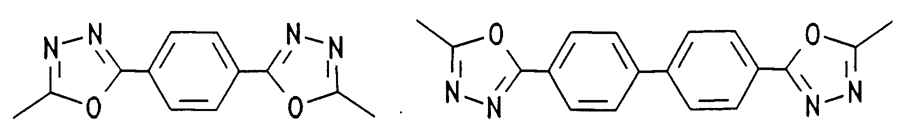 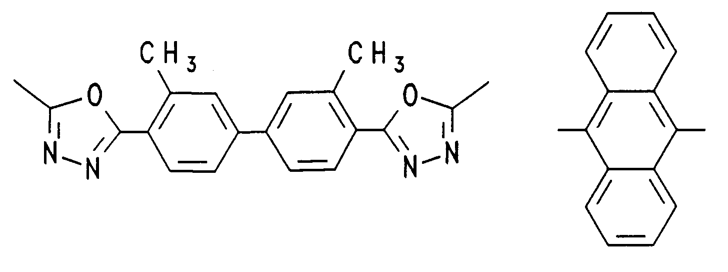
m, n sind gleich oder verschieden 0 oder 1;
C, D sind gleich oder verschieden

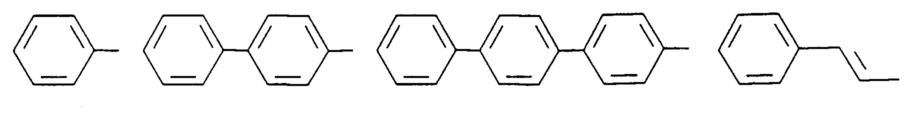

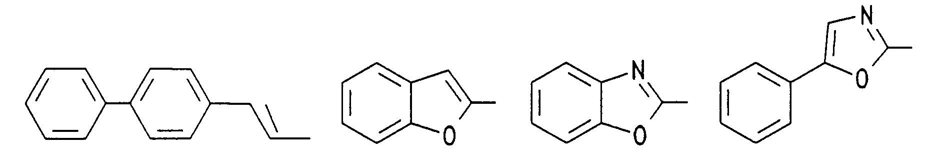

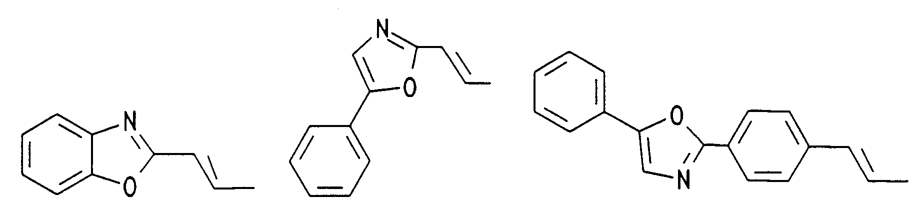

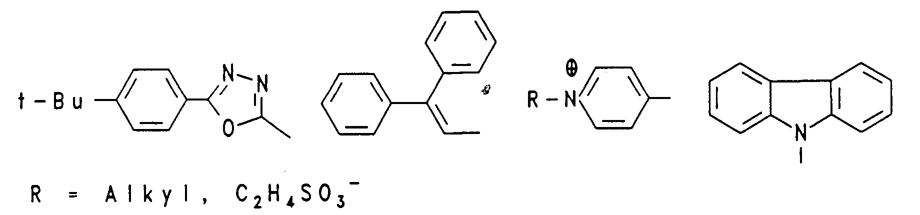

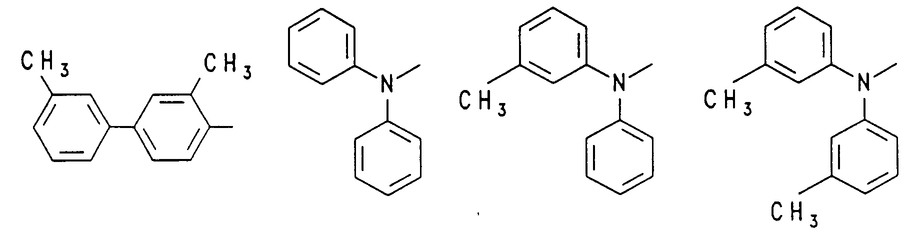

5. Polymer nach einem oder mehreren der vorhergehenden Ansprüche, dadurch gekennzeichnet, daß die Symbole und Indizes in der Formel (I) folgende Bedeutungen haben:
A, B sind gleich oder verschieden 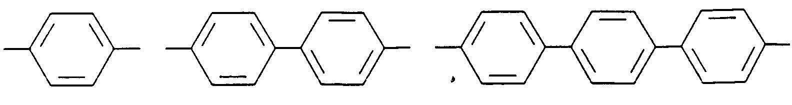 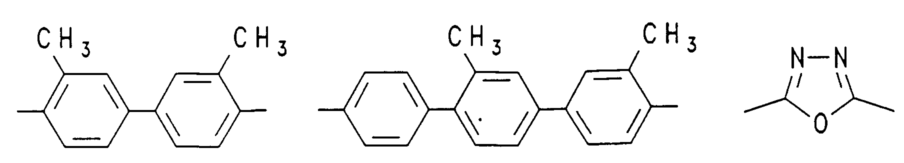 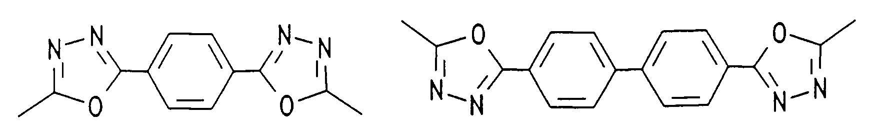 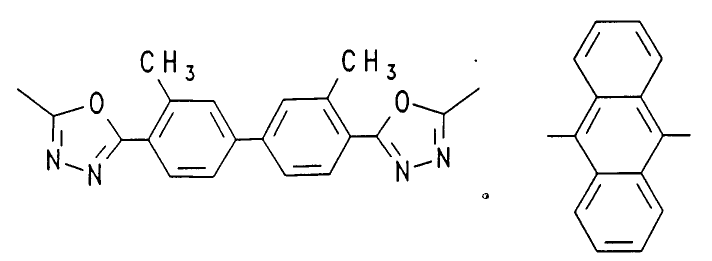
m + n ist 0 oder 1;
C, D sind gleich oder verschieden

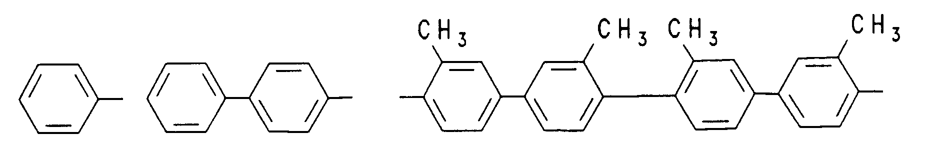

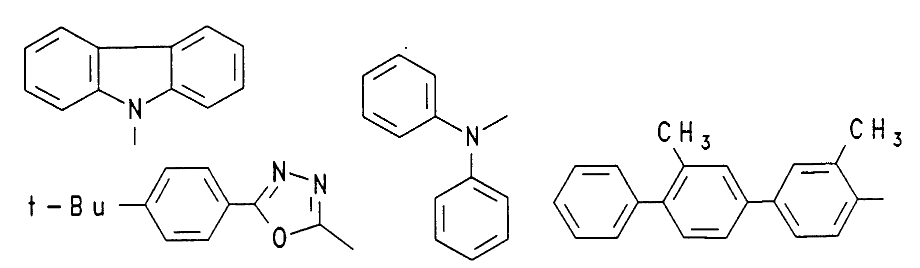

6. Polymer nach einem oder mehreren der vorhergehenden Ansprüche, dadurch gekennzeichnet, daß es ein Copolymer ist.

7. Verwendung eines Polymers nach einem oder mehreren der vorhergehenden Ansprüche als Elektrolumineszenzmaterial.

8. Elektrolumineszenzmaterial, enthaltend ein Polymer nach einem oder mehreren der Ansprüche 1 bis 6.

9. Verfahren zur Herstellung eines Elektrolumineszenzmaterials, dadurch gekennzeichnet, daß ein Polymer nach einem oder mehreren der Ansprüche 1 bis 6 in Form eines Films auf ein Substrat aufgebracht wird.

10. Elektrolumineszenzvorrichtung mit einer oder mehreren aktiven Schichten, dadurch gekennzeichnet, daß mindestens eine dieser aktiven Schichten ein Polymer gemäß einem oder mehreren der Ansprüche 1 bis 6 als Elektrolumineszenzmaterial enthält.

## Claims

1. A conjugated polymer comprising recurring units of the formula (I), 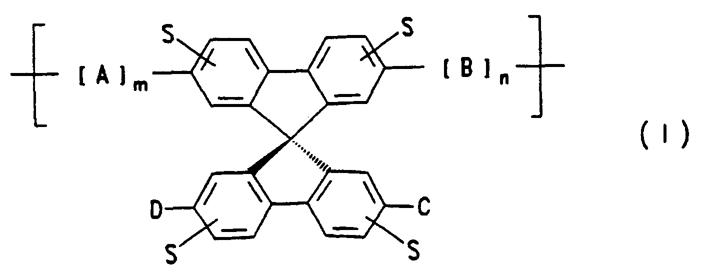 where the symbols and indices have the following meanings:
A, B are identical or different and are each from one to fifteen identical or different arylene and/or heteroarylene and/or vinylene groups which, like the spirobifluorene skeleton itself, may be unsubstituted or substituted;
C, D are identical or different and are each from one to fifteen identical or different arylene and/or heteroarylene and/or vinylene groups which, like the spirobifluorene skeleton itself, may be unsubstituted or substituted, or are hydrogen
S are identical or different and are H or a substituent;
m, n are 0 or 1.

2. A polymer as claimed in claim 1, having from 2 to 1000 recurring units.

3. A polymer as claimed in claim 1 and/or 2, wherein the symbols and indices in the formula (I) have the following meanings:
S are identical or different and are R¹, R², R³ and/or R⁴;
A, B are identical or different and are 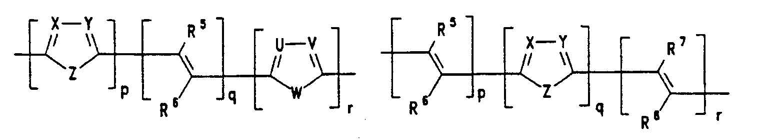 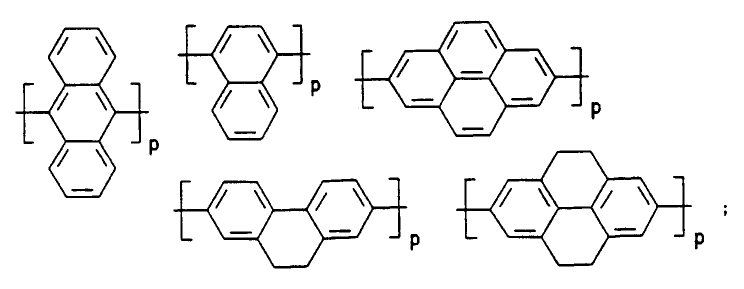
X, Y, U, V are identical or different and are CR⁵, N;
Z, W are identical or different and are -O-, -S-, -NR⁵-, -CR⁵R⁶-, -CR⁵=CR⁶-, -CR⁵=N-;
p, q, r are identical or different and are 0, 1 to 5;
R¹, R², R³, R⁴, R⁵, R⁶, R⁷, R⁸ are identical or different and are H, a straight-chain or branched alkyl, alkoxy or ester group having from 1 to 22 carbon atoms, aryl and/or aryloxy groups, where the aromatic can be substituted by C₁-C₂₂-alkyl, C₁-C₂₂-alkoxy, Br, Cl, F, CN, and/or NO₂, Br, Cl, F, CN, NO₂, CF₃.

4. A polymer as claimed in one or more of the preceding claims, wherein the symbols and indices in the formula (I) have the following meanings:
A, B are identical or different and are 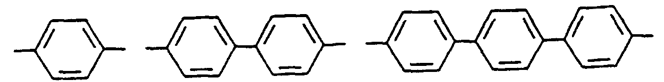 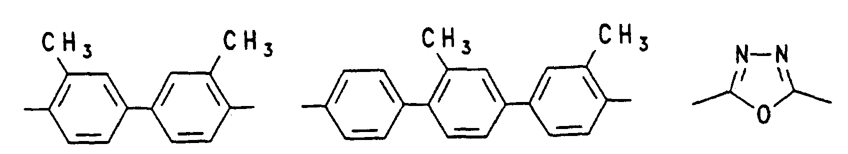 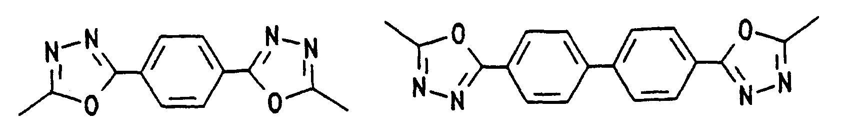 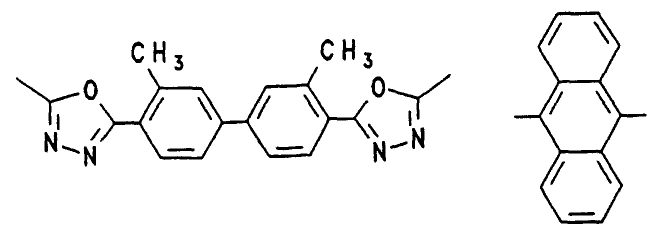
m, n are identical or different and are 0 or 1;
C, D are identical or different and are

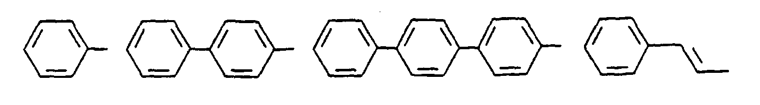

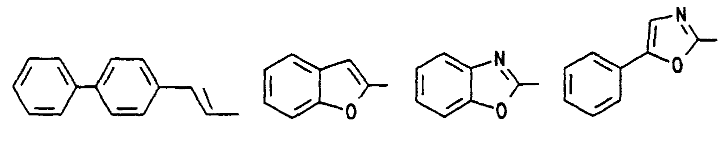

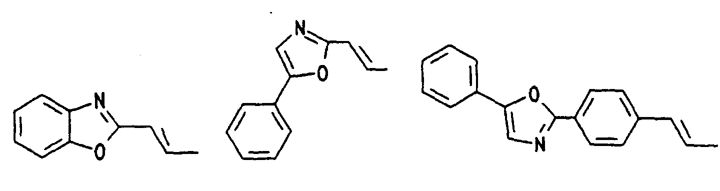

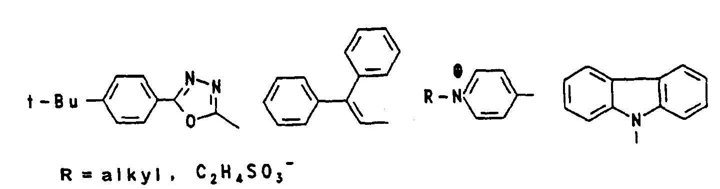

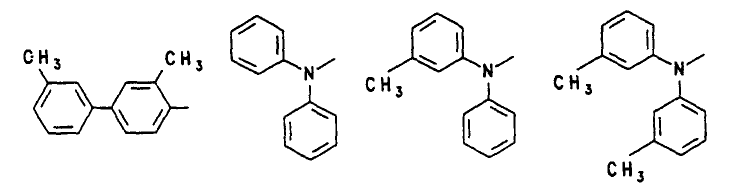

5. A polymer as claimed in one or more of the preceding claims, wherein the symbols and indices in the formula (I) have the following meanings:
A, B are identical or different and are 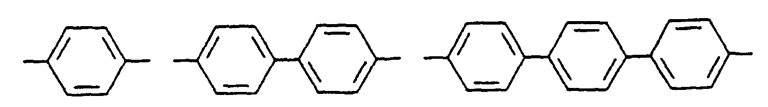 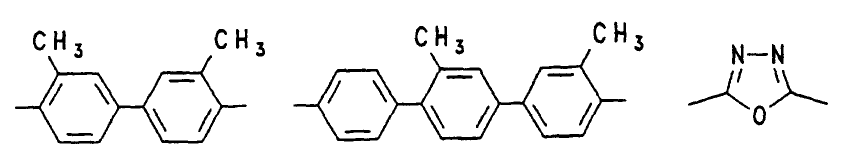 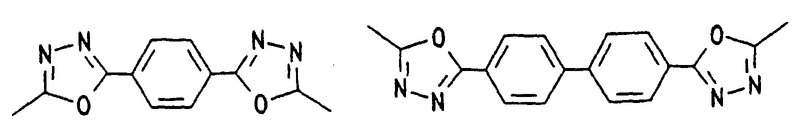 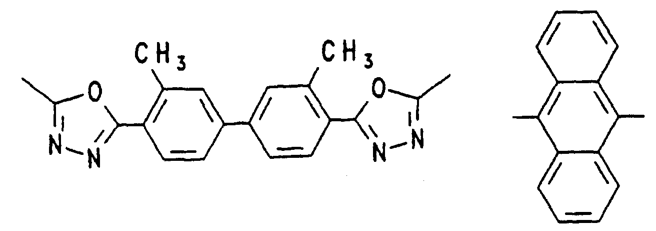
m + n is 0 or 1;
C, D are identical or different and are

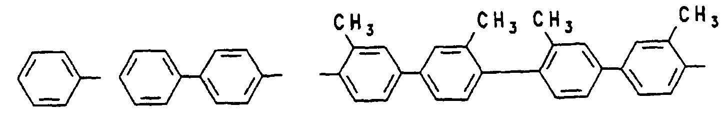

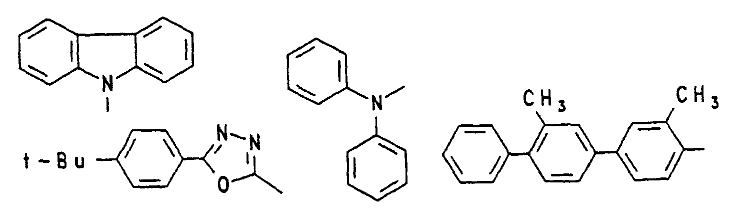

6. A polymer as claimed in one or more of the preceding claims, which is a copolymer.

7. The use of a polymer as claimed in one or more of the preceding claims as an electroluminescence material.

8. An electroluminescence material comprising a polymer as claimed in one or more of claims 1 to 6.

9. A process for producing an electroluminescence material, which comprises applying a polymer as claimed in one or more of claims 1 to 6 in the form of a film to a substrate.

10. An electroluminescence device comprising one or more active layers, wherein at least one of these active layers comprises a polymer as claimed in one or more of claims 1 to 6 as electroluminescence material.

## Revendications

1. Polymère conjugué, contenant des motifs de répétition de formule (I), 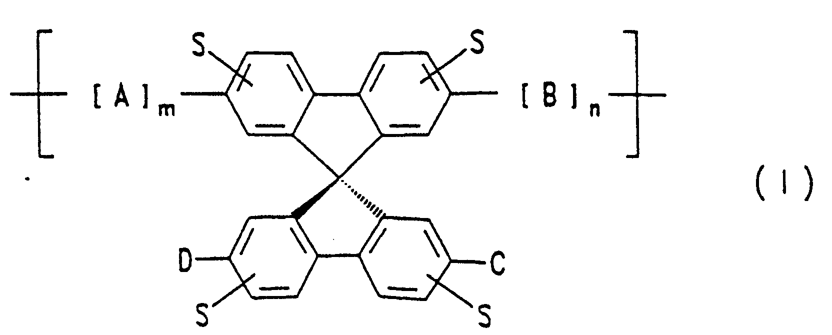 dans laquelle les symboles et indices ont les significations suivantes :
A, B sont identiques ou différents et représentent respectivement de un à quinze groupes arylène et/ou hétéroarylène et/ou vinylène identiques ou différents, qui, de même que le squelette spirobifluorène, peuvent être éventuellement substitués;
C, D sont identiques ou différents et représentent respectivement de un à quinze groupes arylène et/ou hétéroarylène et/ou vinylène identiques ou différents, qui, de même que le squelette spirobifluorène, peuvent être éventuellement substitués, ou un atome d'hydrogène.
S sont identiques ou différents et représentent un atome d'hydrogène ou un substituant;
m, n sont 0 ou 1.

2. Polymère selon la revendication 1, caractérisé en ce qu'il présente de 2 à 1000 motifs de répétition.

3. Polymère selon la revendication 1 et/ou 2, caractérisé en ce que les symboles et indices dans la formule générale (I) ont les significations suivantes :
S est identique ou différent à R¹, R², R³ et/ou R⁴;
A, B sont identiques ou différents et représentent 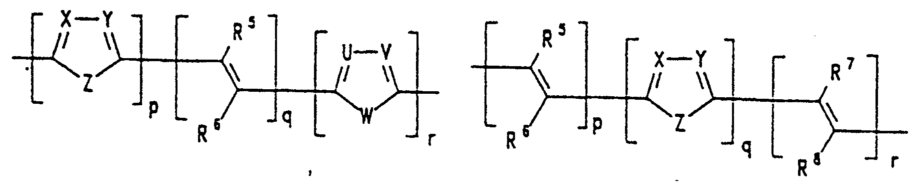 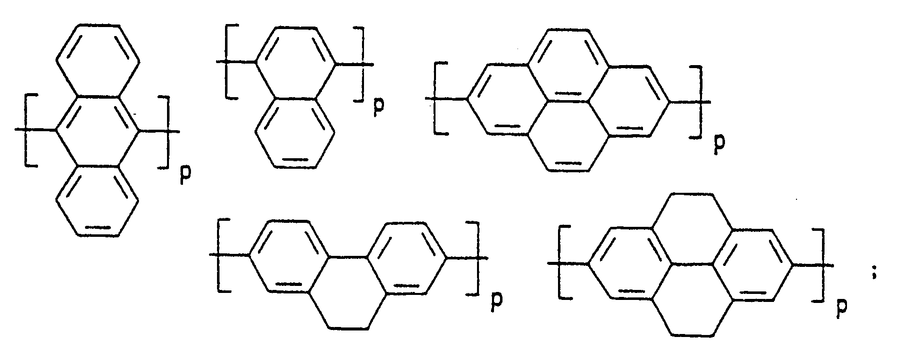 formules dans lesquelles,
X, Y, U, V sont identiques ou différents et représentent CR⁵, N;
Z, W sont identiques ou différents et représentent -O-, -S-, -NR⁵-, -CR⁵R⁶-, -CR⁵=CR⁶-, -CR⁵=N-;
p, q, r sont identiques ou différents et représentent 0, 1 à 5;
R¹, R², R³, R⁴, R⁵, R⁶, R⁷, R⁸ sont identiques ou différents et représentent un atome d'hydrogène, un groupe alkyle, alcoxy ou ester linéaire ou ramifié ayant de 1 à 22 atomes de carbone, des groupes aryle et/ou aryloxy, dans lesquels le groupe aromatique peut être substitué par un groupe alkyle en C₁-C₂₂, alcoxy en C₁-C₂₂, Br, Cl, F, CN, et/ou NO₂, représentent un atome de brome, de chlore, de fluor, un groupe CN, NO₂, CF₃.

4. Polymère selon une ou plusieurs des revendications précédentes, caractérisé en ce que, les symboles et indices de la formule (I) ont les significations suivantes;
A et B sont identiques ou différents et représentent 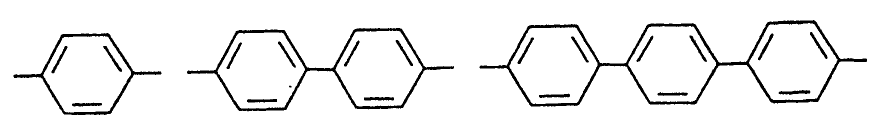 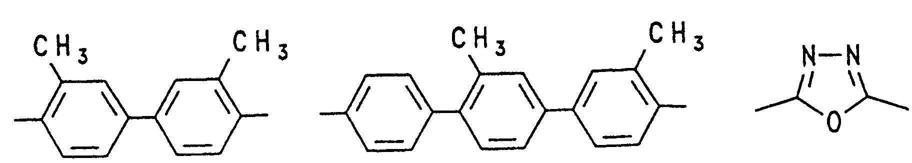 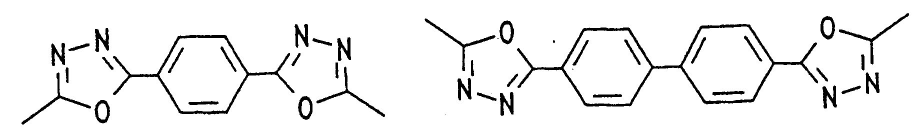 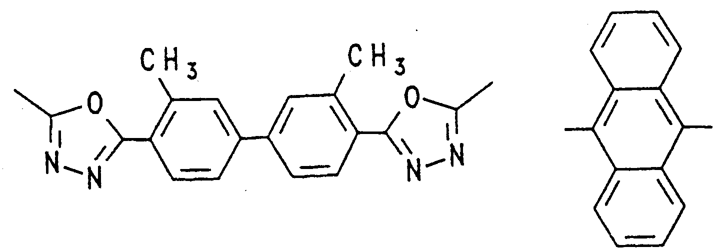
m, n sont identiques ou différents et représentent 0 ou 1;
C, D sont identiques ou différents et représentent

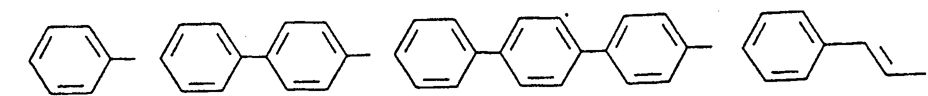

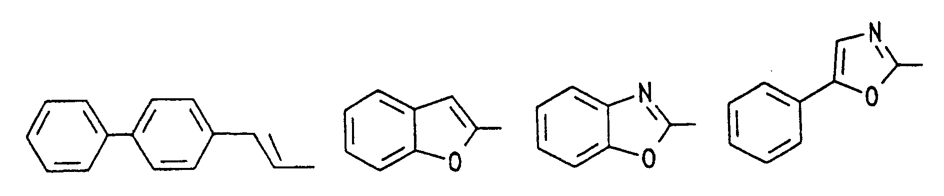

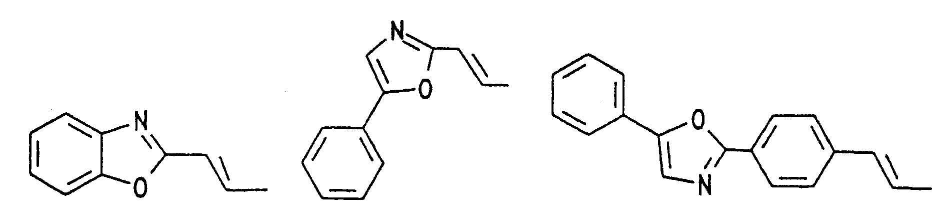

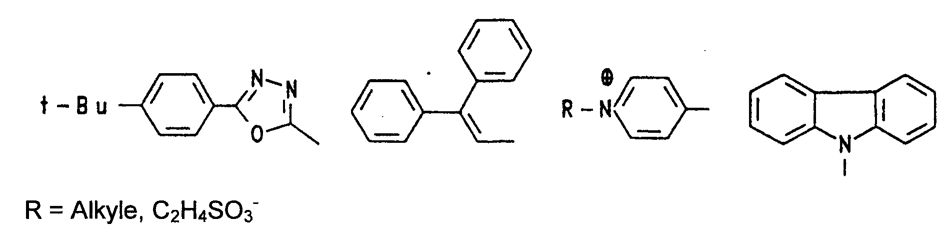

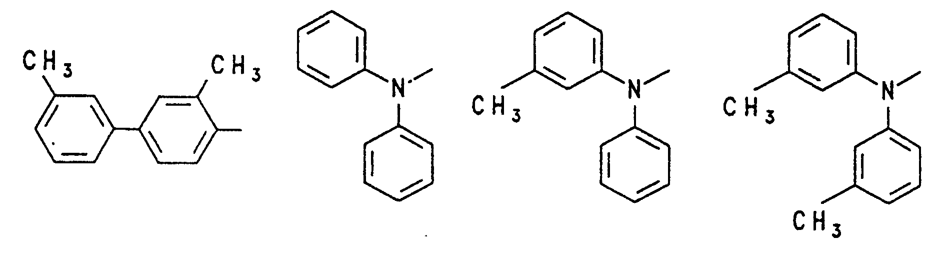

5. Polymère selon une ou plusieurs des revendications précédentes, caractérisé en ce que les symboles et indices ont dans la formule (I) les significations suivantes :
A, B sont identiques ou différents et représentent 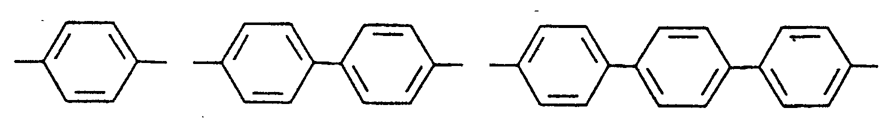 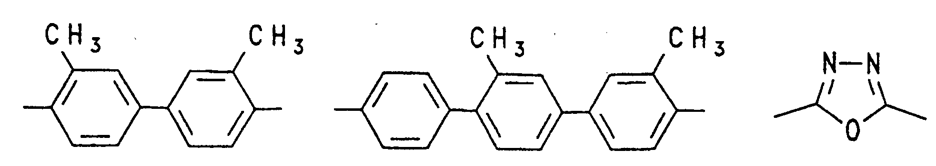 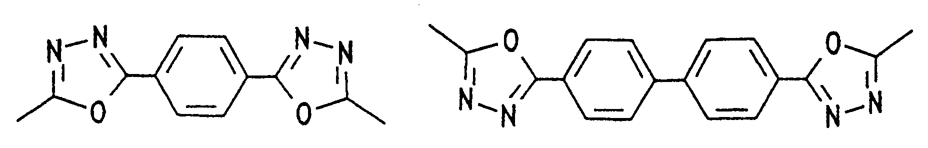 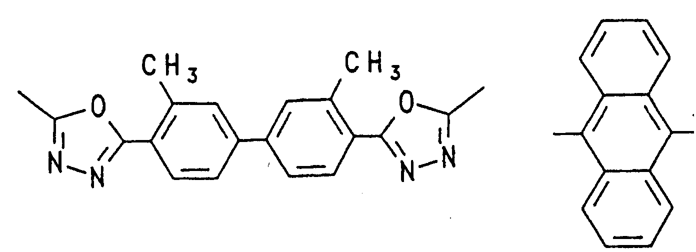
m + n est 0 ou 1;
C, D sont identiques ou différents et représentent

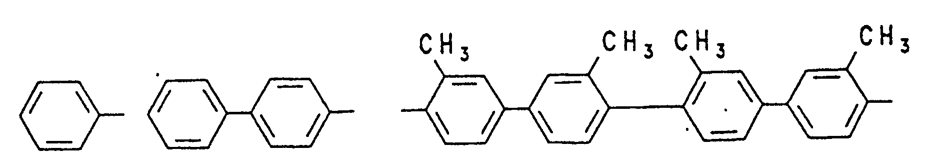

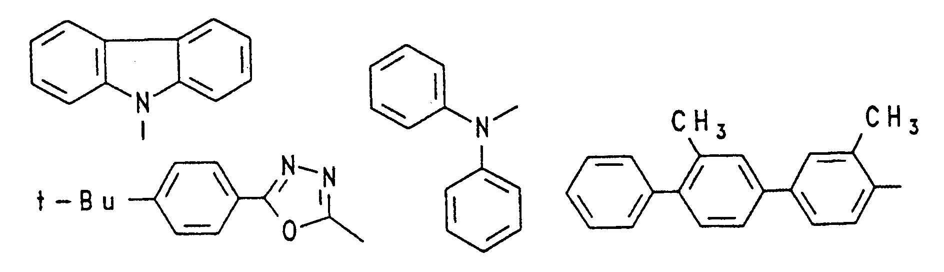

6. Polymère selon une ou plusieurs des revendications précédentes, caractérisé en ce qu'il est un copolymère.

7. Utilisation d'un polymère selon une ou plusieurs des revendications précédentes comme matériau électroluminescent.

8. Matériau électroluminescent, contenant un polymère selon une ou plusieurs de revendications 1 à 6.

9. Procédé pour la préparation d'un matériau électroluminescent, caractérisé en ce qu'on dépose un polymère selon une ou plusieurs de revendications 1 à 6 sur un substrat sous la forme d'un film.

10. Dispositif électroluminescent avec une ou plusieurs couches actives, caractérisé en ce qu'au moins une de ces couches actives contient comme matériau électroluminescent un polymère selon une ou plusieurs des revendications 1 à 6.
